# EUROPEAN PATENT SPECIFICATION

(11) **EP 1 833 788 B1**
(45) Date of publication and mention of the grant of the patent: **16.04.2008**
(21) Application number: 05818419.3
(22) Date of filing: 15.12.2005
(51) Int. Cl.: C07D 209/42, A61K 31/404, A61P 9/12, C07K 5/02

(54) **PROCESS FOR PREPARING PERINDOPRIL ERBUMINE**
VERFAHREN ZUR HERSTELLUNG VON PERINDOPRILERBUMIN
PROCÉDÉ DE PREPARATION DE PERINDOPRIL ERBUMINE

(30) Priority: 31.12.2004 ES 200403168
(43) Date of publication of application: 19.09.2007
(73) Proprietor: Quimica Sintetica, S.A., 08028 Barcelona (ES)
(72) Inventor: PALOMO NICOLAU, Francisco, E-28804 Alcalà de Henares (ES); DE LEON, Dorcas, E-28804 Alcalà de Henares (ES)
(74) Representative: Ponti Sales, Adelaida
(86) International application number: PCT/IB2005/003928
(87) International publication number: WO 2006/070276

(56) References cited:
- EP-A- 0 308 341
- EP-A- 1 333 026
- WO-A-03/010142

## Description

### Field of the invention

The present invention discloses a process for producing the perindopril erbumine compound of formula I, useful in the treatment of hypertension.

### State of the prior art

Perindopril erbumine is the international nonproprietary name of the tert-butylamine salt (2S,3aS,7aS)-1-[(2S)-2-[[(1S)-1-(ethoxycarbonyl)butyl] amino]-1-oxopropyl]-octahydro-1-1H-indole-2-carboxylic acid, applicable in medicine and known as an antihypertensive inhibitor of the angiotensin converter enzyme (IAEC).

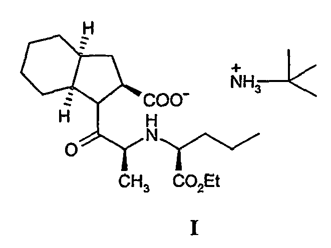

Perindopril was first described in the European patent EP 49.658 B1. In European patent EP 308.341 the perindopril t-butylamine salt was prepared for the first time from Ethyl L-Norvalinate, pyruvic acid and (2S, 3aS, 7aS) octahydroindole-2-carboxylic acid. The acid Perindopril is obtained following hydrogenation of the benzyl ester and freeze-drying the aqueous solution that contains it; finally, the t-butylamine salt is prepared in ethyl acetate.

Patent application WO 03/064388 showed that obtaining the perindopril erbumine salt by the process disclosed in EP 308.341 B1 requires several purification steps in order to obtain a product that meets the specifications required for the preparation of active pharmaceutical ingredients, stating the difficulty of separating out the impurities resulting from the reaction with dicyclohexylcarbodiimide. That document proposed as a better process the preparation of N-(2-ethoxycarbonyl)butyl-N-alkyloxycarbonylalanine, followed by the formation of the acid chloride, subsequent reaction with the (2S, 3aS, 7aS) octahydroindole-2-carboxylic acid and addition of t-butylamine in order to isolate the product. This process nevertheless suffers from the disadvantages of protecting the amino group with alcoxycarbonyl, evaporating the solvent used for the reaction of the peptidic bond and providing a low yield of the erbumine salt (35 to 55%).

Patent application WO 01/58868 A1 disclosed an improved process for obtaining perindopril erbumine by reaction with dicyclohexylcarbodiimide, which nevertheless leads to a product with the impurities resulting from the use of this reactant at levels of 0.2 and 0.1%. The process involves protecting the (2S, 3aS, 7aS) octahydroindole-2-carboxylic acid as benzyl ester, deprotecting the perindopril benzyl ester, freeze-drying the aqueous solution of the acid perindopril, dissolving in ethyl acetate and precipitating the salt by addition of t-butylamine.

Other processes for synthesising perindopril are disclosed in patent applications WO 03/010142 A2 and WO 04/099236 A1. Both are characterised by carrying out the reaction directly with the (2S, 3aS, 7aS) octahydroindole-2-carboxylic acid (Formula II), but require complicated elaborations consisting of extractions and evaporations of solvents, with the danger of forming dicetopiperazine (an impurity of the degradation of dipeptides that occurs at the isoelectric point and at lower pH levels, Formula III), which is increased in industrial batches. The use of phosgene in the first, and of expensive tetramethyluronium salts in the second, constitute a disadvantage for the industrial execution of both methods.

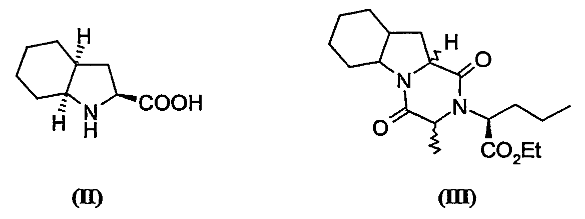

Spanish patent ES 2.156.037 claims the use of soluble amino acid salts in non-aqueous solvents and their use in the preparation of dipeptides. That document disclosed for the first time the reaction of DBU salts and amino acids with oxime esters of N-alkyl amino acids with high yields. Making the reaction mixture involves washing the organic phase with water and pH adjustments, followed by distilling the solvent and isolating the N-alkyldipeptide.

It is therefore necessary to design a robust industrial process to provide the perindopril erbumine salt that avoids the disadvantages mentioned above: the use of expensive and hazardous reactants, complicated elaborations in order to extract the acid perindopril, low yields and the degradation to dicetopiperazine (III).

### Object of the invention

The object of the present invention is a process that provides a simple and inexpensive industrial process for preparing perindopril erbumine in pure form (purity greater than 99.5% determined by HPLC) that comprises reacting the salt formed between the (2S, 3aS, 7aS) octahydroindole-2-carboxylic acid and organic bases with the active ester of N-(ethoxycarbonyl)butyl-L-alanine, and then precipitating the tert-butylamine salt without the need for extracting and purifying the other compounds that take part in the reaction. Since the reaction is carried out directly with the amino acid, two steps are removed from the synthesis: a subsequent deprotection reaction to obtain the product and a step of protection of the amino group for its activation to oxime ester. This process has the following advantages over those described:
- the reaction is carried out directly with the (2S, 3aS, 7aS) octahydroindole-2-carboxylic acid without the need for submitting the product thus obtained to a subsequent deprotection reaction.
- The reaction proceeds with a good yield (88 to 94%)
- Formation and isolation of the product proceed via the salt, thereby avoiding the formation of dicetopiperazine.
- The reactants that take part in the formation reaction do not interfere in the crystallisation of the salt (DBU, acetonoxime), providing a purity greater than 99.5% as measured by HPLC.

### Detailed description of the invention

The object of the present invention is a process for obtaining the 1-[(2S)-2-[[(1S)-1(ethoxycarbonyl)butyl]amino]-1-propionyl] (2S,3aS,7aS)-octahydro-1H-indole-2-carboxylic acid, tert-butylamine salt that comprises reacting an active ester of N-[2(S)-(ethoxycarbonyl)butyl]-L-alanine with an organic salt of perhydroindole-2-carboxylic acid, followed by the addition of t-butylamine and an acid to the reaction medium, in any order, or else a salt formed from an acid and tert-butylamine and then separating the product by conventional processes.

The starting material N-(2-ethoxycarbonyl)butyl-L-alanine is described in the European patents EP 0308340B1, EP 0308341B1 and EP309324B1. (2S, 3aS, 7aS) octahydroindole-2-carboxylic acid is described in the European patents EP 0308339B1 and EP 0308341B1.

A solution of (2S, 3aS, 7aS) octahydroindole-2-carboxylic acid (II) is obtained by reaction with an organic base in an organic solvent, such as acetonitrile, methylene chloride, dimethylformamide, dimethylsulphoxide, tetrahydrofuran, within a temperature range from -20 to 50°C. Organic bases that fulfil the dual function of solubilising and activating the imino group include the phosphacines known as the Schwesinger bases, DBU (1,8 Diazabicyclo[5.4.0]undec-7-ene), DBN (1,5-Diazabicyclo[4.3.0]non-5-ene) and substituted guanidines such as tetramethylguanidines.

The reaction of the salt of the acid of formula II to form the perindopril compound is carried out with an active ester of N-(2-ethoxycarbonyl)butyl-L-alanine, such as an acetonoxime ester, a thiolester, nitrophenol ester, cyanomethylester, 2-pyridylketone oxime, prepared as described in patent ES 2.156.037. The reaction takes place over 4 to 24 hours at temperatures of 0 to 100°C, preferably between 30 and 70°C.

Once the reaction has finished, t-butylamine and an inorganic acid or an organic acid, preferably an inorganic acid such as hydrochloric acid, are added in any order. A salt formed from an inorganic acid or an organic acid and tert-butylamine can also be added. Perindopril erbumine is separated by conventional methodes, preferably by precipitation.

One specific embodiment of the present invention comprises the reaction of the DBU salt of (2S, 3aS, 7aS) octahydroindole-2-carboxylic acid dissolved in acetonitrile with the acetonoxime ester of N-(2-ethoxycarbonyl)butyl-L-alanine at 50°C. Once the reaction has finished, t-butylamine and hydrochloric acid or tert-butylamine hydrochloride are added in any order. The erbumine salt thus obtained presents a purity greater than 99.5% and a yield higher than 85%.

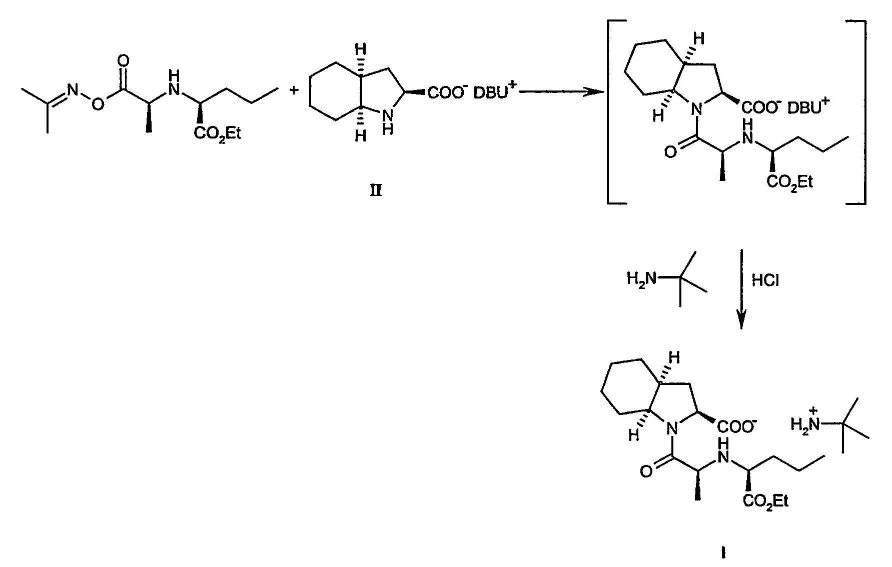

The following examples illustrate the invention, but should not be considered as restricting the scope thereof.

### Example 1: N(S)-[2-(ethoxycarbonyl)butyl]-L-alanine ester of acetonoxime

### Part A: Vilsmeier Reactant

8.11 g (110 mmoles) of N,N-Dimethylformamide are dissolved in a mixture of 8.1 ml of dioxane and 42 ml of methylene chloride, under nitrogen atmosphere, and the solution is cooled down to 0-5° C. Hydrogen chloride gas is bubbled through the solution and then 13.7 g (108 mmoles) of oxalyl chloride are added. The mixture is kept under stirring at between 0-5 ° C for 1 hour.

### Part B: Reaction

20 g (92.4 mmoles) of N-[2(S)-(ethoxycarbonyl)butyl]-L-alanine are suspended in a mixture of 1.6 ml of dioxane and 32 ml of methylene chloride, in nitrogen atmosphere. The mixture is cooled to 0-5° C and hydrogen chloride gas is bubbled through it. The Vilsmeier reactant obtained in part A is immediately added and after one hour of stirring, 8.1 g (110 mmoles) of acetonoxime are added. Once the reaction has finished, the mixture is extracted with 150 ml of potassium carbonate in water (20%) and 300 ml of water; the resulting organic phase is dried over anhydrous sodium sulphate and concentrated in vacuo to give 22.6 g of a yellowish oil (Yield: 95% of the theoretical).

### Example 2: 1-[(2S)-2-[[(1S)-1(ethoxycarbonyl)butyl]amino]-1-propionyl] (2S,3aS,7aS)-octahydro-1H-indole-2-carboxylic acid, tert-butylamine salt

9.5 g (56.7 mmoles) of (2S, 3aS, 7aS) octahydroindole 2-carboxylic acid are suspended in 17.3 ml (56.7 mmoles) of P₁-t-Bu-tris(tetramethylene) (phosphacene) and 30 ml of acetonitrile, in nitrogen atmosphere. 16.2 g of N(S)-[2-(ethoxycarbonyl)butyl]-L-alanine ester of acetonoxime (obtained according to Example 1) are added, and the mixture is heated at 50°C until the reaction is completed. The acetonitrile is then concentrated, and the residue obtained is dissolved in water and washed with isopropyl acetate; the aqueous phase is adjusted to pH 4-5 with hydrochloric acid (35%) and extracted with 150 ml of methylene chloride. The organic phase is cooled to 0-10° C and 5.9 ml (56.7 mmoles) of tert-butylamine are added to it. The solution is stirred for half an hour and concentrated in vacuo to give 22.5 g of a white solid of 99.5% purity. (Yield: 90% of the theoretical).

### Example 3: 1-[(2S)-2-[[(1S)-1(ethoxycarbonyl)butyl]amino]-1-propionyl] (2S, 3aS, 7aS)-octahydro-1H-indole-2-carboxylic acid, tert-butylamine salt

9.5 g (56.7 mmoles) of (2S, 3aS, 7aS) octahydroindole 2-carboxylic acid are suspended in 8.6 ml (56.7 mmoles) of 1,8 Diazabicyclo[5.4.0]undec-7-ene (DBU) and 30 ml of acetonitrile, under nitrogen atmosphere, and the mixture is stirred until total dissolution. 16.2 g of [L-Norvaline, N-[(1S)-1-carboxyethyl]-1-ethyl ester of acetonoxime] (obtained according to Example 1) are added, and the mixture is heated at 40-45° C until the reaction is completed. The reaction mixture is diluted with more acetonitrile (300 ml), is heated to 50-60° C and 5.9 ml of tert-butylamine and 2.1 g of hydrogen chloride gas are added. It is cooled to 0-10° C, and the precipitate is filtered and washed with acetonitrile (2 x 25 ml). The product obtained is dried in a vacuum oven at 40°C to give 22 g of a white solid of 99.5% purity. (Yield: 90% of the theoretical).

## Claims

1. Process for obtaining 1-[(2S)-2-[[(1S)-1(ethoxycarbonyl)butyl]amino]-1-propionyl] (2S,3aS,7aS)-octahydro-1H-indole-2-carboxylic acid, tert-butylamine salt, which comprises reacting an active ester of N-[2(S)-(ethoxycarbonyl)butyl]-L-alanine with an organic salt of perhydroindole-2-carboxylic acid, followed by the addition of t-butylamine and an acid to the reaction medium, in any order, or else a salt formed from an acid and tert-butylamine, and then separating the product by conventional methods.

2. Process according to claim 1, **characterised in that** the organic salt of perhydroindole-2-carboxylic acid is a salt obtained by reaction of (2S, 3aS, 7aS) octahydroindole-2-carboxylic acid (II) with an organic base that fulfils the dual function of solubilising and activating the imino group, such as the phosphacenes (Schwesinger bases), DBU (1,8 Diazabicyclo[5.4.0]undec-7-ene), DBN (1,5-Diazabicyclo[4.3.0]non-5-ene) and substituted guanidines such as tetramethylguanidines.

3. Process according to claim 2, **characterised in that** the reaction between perhydroindole-2-carboxylic acid and the organic base is carried out in an organic solvent, such as acetonitrile, methylene chloride, dimethylformamide, dimethylsulphoxide or tetrahydrofuran, within a temperature range between -20 and 50°C.

4. Process according to claim 1, **characterised in that** the active ester of N-(2-ethoxycarbonyl)butyl-L-alanine is selected from an ester of acetonoxime, a thiolester, nitrophenol ester, cyanomethylester or 2-pyridylketone oxime.

5. Process according to claim 1, **characterised in that** the reaction between the active ester of N-[2(S)-(ethoxycarbonyl)butyl]-L-alanine and the organic salt of the perhydroindole-2-carboxylic acid is carried out over 4 to 24 hours at temperatures from 0 to 100°C, preferably between 30 and 70°C.

6. Process according to claim 1, **characterised in that** t-butylamine and an inorganic acid or an organic acid, preferably an inorganic acid, are added in any order, or else a salt formed from an inorganic acid or organic acid and tert-butylamine is added, and the perindopril erbumine is separated by conventional methods, preferably by precipitation.

7. Process according to claim 1, **characterised in that** it comprises reaction of the DBU salt of (2S, 3aS, 7aS) octahydroindole-2-carboxylic acid dissolved in acetonitrile with the acetonoxime ester of N-(2-ethoxycarbonyl)butyl-L-alanine at 50°C, followed by the addition in any order of t-butylamine and hydrochloric acid or of tert-butylamine hydrochloride.

8. Process according to claim 1, **characterised in that** no racemisation arises, obtaining the stereoisomer SSSSS.

9. Process according to claim 1, **characterised in that** perindopril erbumine is obtained with a purity greater than 99.5% as determined by HPLC.

## Patentansprüche

1. Verfahren zur Herstellung von 1-[(2S)-2-[[(1S)-1(Ethoxycarbonyl)butyl]amino-1-propionyl] (2S, 3aS, 7aS)-octahydro-1H-indol-2-carbonsäure, tert-Butylaminsalz, umfassend die Umsetzung eines aktiven Esters von N-[2(S)-(Ethoxycarbonyl)butyl]-L-alanin mit einem organischen Salz der Perhydroindol-2-carbonsäure, gefolgt von der Zugabe von t-Butylamin und einer Säure zum Reaktionsmedium, in beliebiger Reihenfolge, oder ansonsten eines Salzes, gebildet aus einer Säure und tert-Butylamin, und anschließendes Abtrennen des Produkts durch übliche Verfahren.

2. Verfahren nach Anspruch 1, **dadurch gekennzeichnet, dass** das organische Salz der Perhydroindol-2-carbonsäure ein Salz ist, erhalten durch die Umsetzung von (2S, 3aS, 7aS) Octahydroindol-2-carbonsäure (II) mit einer organischen Base, die die doppelte Funktion der Solubilisierung und Aktivierung der Iminogruppe erfüllt, beispielsweise die Phosphazene (Schwesinger Basen), DBU (1,8 Diazabicyclo[5.4.0]undec-7-en), DBN (1,5-Diazabicyclo[4.3.0]non-5-en] und substituierte Guanidine wie Tetramethylguanidine.

3. Verfahren nach Anspruch 2, **dadurch gekennzeichnet, dass** die Umsetzung zwischen Perhydroindol-2-carbonsäure und der organischen Base in einem organischen Lösungsmittel durchgeführt wird, beispielsweise Acetonitril, Methylenchlorid, Dimethylformamid, Dimethylsulfoxid oder Tetrahydrofuran, innerhalb eines Temperaturbereichs von -20 bis 50°C.

4. Verfahren nach Anspruch 1, **dadurch gekennzeichnet, dass** der aktive Ester N-(2-Ethoxycarbonyl)butyl-L-alanin ausgewählt wird aus einem Ester von Acetonoxim, einem Thiolester, Nitrophenolester, Cyanomethylester oder 2-Pyridylketonoxim.

5. Verfahren nach Anspruch 1, **dadurch gekennzeichnet, dass** die Umsetzung zwischen dem aktiven Ester von N-[2(S)-(Ethoxycarbonyl)butyl]-L-alanin und dem organischen Salz der Perhydroindol-2-carbonsäure über 4 bis 24 Stunden bei Temperaturen von 0 bis 100°C, bevorzugt zwischen 30 und 70°C, durchgeführt wird.

6. Verfahren nach Anspruch 1, **dadurch gekennzeichnet, dass** t-Butylamin und eine anorganische Säure oder eine organische Säure, bevorzugt eine anorganische Säure, in beliebiger Reihenfolge zugegeben werden, oder ansonsten ein Salz aus einer anorganischen Säure oder einer organischen Säure und tert-Butylamin zugegeben wird, und dass Perindoprilerbumin durch übliche Verfahren, bevorzugt durch Präzipitation, abgetrennt wird.

7. Verfahren nach Anspruch 1, **dadurch gekennzeichnet, dass** es die Umsetzung des DBU-Salzes von (2S, 3aS, 7aS) Octahydroindol-2-carbonsäure, gelöst in Acetonitril, mit dem Acetonoximester von N-(2-Ethoxycarbonyl)butyl-L-alanin bei 50°C umfasst, gefolgt durch die Zugabe, in beliebiger Reihenfolge, von t-Butylamin und Hydrochlorsäure oder von tert-Butylaminhydrochlorid.

8. Verfahren nach Anspruch 1, **dadurch gekennzeichnet, dass** keine Racemisierung auftritt, wodurch das Stereoisomer SSSSS erhalten wird.

9. Verfahren nach Anspruch 1, **dadurch gekennzeichnet, dass** Perindoprilerbumin in einer Reinheit von über 99,5%, bestimmt durch HPLC, erhalten wird.

## Revendications

1. Procédé d'obtention du sel de tert-butylamine de l'acide 1-[(2S)-2-[[(1S)-1(éthoxycarbonyl)butyl]amino]-1-propionyl] (2S, 3aS, 7aS)-octahydro-1H-indole-2-carboxylique, comprenant la réaction d'un ester activé de la N-[2(S)-(éthoxycarbonyl)butyl]-L-alanine avec un sel organique de l'acide perhydroindole-2-carboxylique, suivie de l'addition de t-butylamine et d'un acide au milieu réactionnel, dans un ordre quelconque, ou sinon d'un sel formé à partir d'un acide et de tert-butylamine, et ensuite la séparation du produit selon des procédés conventionnels.

2. Procédé selon la revendication 1, **caractérisé en ce que** le sel organique de l'acide perhydroindole-2-carboxylique est un sel obtenu par réaction de l'acide (2S, 3aS, 7aS)-octahydroindole-2-carboxylique (II) avec une base organique qui rempli la double fonction de solubiliser et d'activer le groupe imino, telle que les phosphacènes (bases de Schwesinger), le DBU (1,8-diazabicyclo[5.4.0]undéc-7-ène), le DBN (1,5-diazabicyclo[4.3.0]non-5-ène) et des guanidines substituées telles que les tétraméthylguanidines.

3. Procédé selon la revendication 2, **caractérisé en ce que** la réaction entre l'acide perhydroindole-2-carboxylique et la base organique est effectuée dans un solvant organique, tel que l'acétonitrile, le chlorure de méthylène, le diméthylformamide, le diméthylsulfoxyde ou le tétrahydrofurane, à une température comprise dans la plage de -20 à 50°C.

4. Procédé selon la revendication 1, **caractérisé en ce que** l'ester activé de la N-(2-éthoxycarbonyl)butyl-L-alanine est choisi parmi un ester d'acétonoxime, un thiolester, le nitrophénol ester, le cyanométhylester ou la 2-pyridylcétone oxime.

5. Procédé selon la revendication 1, **caractérisé en ce que** la réaction entre l'ester activé de la N-[2(S)-(éthoxycarbonyl)butyl]-L-alanine et le sel organique de l'acide perhydroindole-2-carboxylique est effectuée en 4 à 24 heures à une température de 0 à 100°C, de préférence entre 30 et 70°C.

6. Procédé selon la revendication 1, **caractérisé en ce que** la t-butylamine et un acide inorganique ou un acide organique, de préférence un acide inorganique, sont ajoutés dans un ordre quelconque, ou sinon un sel formé à partir d'un acide inorganique ou d'un acide organique et de tert-butylamine est ajouté, et le périndopril erbumine est séparé selon des procédés conventionnels, de préférence par précipitation.

7. Procédé selon la revendication 1, **caractérisé en ce qu'**il comprend la réaction entre le sel de DBU de l'acide (2S, 3aS, 7aS)-octahydroindole-2-carboxylique dissous dans l'acétonitrile avec l'ester d'acétonoxime de la N-(2-éthoxycarbonyl)butyl-L-alanine à 50°C, suivie de l'addition dans un ordre quelconque de t-butylamine et d'acide chlorhydrique ou de chlorhydrate de tert-butylamine.

8. Procédé selon la revendication 1, **caractérisé en ce qu'**il ne se produit pas de racémisation, ce qui donne le stéréoisomère SSSSS.

9. Procédé selon la revendication 1, **caractérisé en ce que** le périndopril erbumine est obtenu avec une pureté supérieure à 99,5% telle que déterminée par HPLC.
